# EUROPEAN PATENT SPECIFICATION

(11) **EP 1 468 677 B1**
(45) Date of publication and mention of the grant of the patent: **08.08.2012**
(21) Application number: 04425213.8
(22) Date of filing: 25.03.2004
(51) Int. Cl.: A61K 9/10, A61K 31/734, A61K 36/66, A61K 36/185

(54) **An antireflux syrup**
Sirupzubereitung gegen Sodbrennen
Préparation de sirop contre le pyrosis

(30) Priority: 03.04.2003 IT RM20030154
(43) Date of publication of application: 20.10.2004
(73) Proprietor: D.M.G. Italia Srl, 00040 Pomezia (RM) (IT)
(72) Inventor: Mercuri, Luigi, 00181 Roma (IT)
(74) Representative: Sarpi, Maurizio

(56) References cited:
- WO-A-00/67799
- GB-A- 2 298 365
- GB-A- 2 324 724
- US-B1- 6 348 502
- DATABASE BIOSIS [Online] BIOSCIENCES INFORMATION SERVICE, PHILADELPHIA, PA, US; 1991, ING A J ET AL: "CHRONIC PERSISTENT COUGH AND GASTRO-ESOPHAGEAL REFLUX" XP002294423 Database accession no. PREV199192093850 & THORAX, vol. 46, no. 7, 1991, pages 479-483, ISSN: 0040-6376
- DATABASE BIOSIS [Online] BIOSCIENCES INFORMATION SERVICE, PHILADELPHIA, PA, US; 2002, IRWIN RICHARD S ET AL: "Diagnosis and treatment of chronic cough due to gastro-esophageal reflux disease and postnasal drip syndrome" XP002294424 Database accession no. PREV200200469242 & PULMONARY PHARMACOLOGY AND THERAPEUTICS, vol. 15, no. 3, 2002, pages 261-266, ISSN: 1094-5539
- DATABASE MEDLINE [Online] US NATIONAL LIBRARY OF MEDICINE (NLM), BETHESDA, MD, US; June 1995 (1995-06), NISHI K ET AL: "[A case of chronic persistent cough caused by gastro-esophageal reflux]" XP002294425 Database accession no. NLM7666622 & NIHON KYOBU SHIKKAN GAKKAI ZASSHI. JUN 1995, vol. 33, no. 6, June 1995 (1995-06), pages 652-659, ISSN: 0301-1542

## Description

This invention relates to the pharmaceutical field, and more precisely it relates to a mechanically acting medical device which is fit for treatment of gastric reflux and is in the form of a syrup, said syrup containing silicone oils, magnesium alginates, carboxypolymethylene, re-epithelization compounds and sedative compounds. Said medical composition is able to reduce symptoms and troubles whose main cause is gastric reflux, as for instance: cough, pyrosis (sensation of burning at the epigastrium with spreading up to the oesophagus and the pharynx), oesophagitis, dysphonias, rhinopharyngeal phlogoses, disepithelization and regurgitations; moreover, it exerts a coadjuvant action in re-epithelization processes.

It is an object of this invention to supply a composition in the form of a syrup which is capable of reducing the gastric reflux, of limiting the damages caused by said reflux so as to reduce also its incidence and to limit the use of pharmacological compounds with undesired secondary effects.

The gastroesophageal reflux disease (known as GERD from GastroEsophageal Reflux Disease) is a pathology which for instance in Italy concerns one third of the whole population, so that it is increasingly drawing the interest of scientific community.

The pathogenesis of the GERD is to be ascribed to changes in the oesophageal defense mechanisms together with an increased activity of aggressive factors. The oesophageal defense mechanisms consist in the antireflux barrier, which controls the events and the volume of the refluxed matter, in the luminal clearance, which shortens the holdup time of the refluxed matter within the oesophagus, and in the factors of epithelial resistance which are capable of limiting the damage during the contact of the acid with the mucous membrane. The antireflux barrier function stems from an optimal anatomico-physiological equilibrium. The pathological reflux can thus be a side issue of conditions of lowered efficiency of the containment mechanism supplied by the lower oesophageal sphincter, of the increase of intragastric pressure, of troubles in the gastroesophageal mobility as well as of other factors of neurohormonal or pharmacological nature. The presence of slide-originated hiatal hernia can increase the number of episodes as well as the amount of refluxed matter.

Clearance occurs through primary and secondary peristalsis, thanks to which the passing of saliva into the oesophagus, with consequent blocking effect, and the prokinetic cleaning action which takes the refluxed matter again toward stomach are made possible.

Damages from oesophageal reflux are also limited by resistance factors, as for instance the mucus layer, saliva and glandular aqueous and alkaline excretion, as well as by epithelium with its intercellular junctions.

Independently of the pathological responsible mechanism there are conditions that can favour the appearance of gastroesophageal reflux. Among such conditions there are the following ones: the habit of having square meals, of lying down immediately after having meals, of wearing clothes too narrow at the waist, of eating some foods or drinking alcoholic drinks and gas-charged beverages, of smoking, and the condition of being overweight (which causes an increase in abdominal pressure). Moreover, some classes of drugs like calcium-antagonists, theophylline compounds, beta-2-agonists and anti-parkinson drugs show facilitating actions.

The typical symptoms of gatroesophageal reflux are pyrosis (burning sensation at epigastrium with spreading up to oesophagus and pharynx) and the acid regurgitation that is felt like a liquid which tastes acid and bitter. The GERD can also occur with atypical manifestations that can be classified into oesophageal and extraoesophageal symptoms.

Among the first ones there are phagiodynia (painful swallowing) and dysphagia (difficulty in swallowing). Among the atypical extraoesophageal symptoms there are anginous-like thorax pain, chronic laryngitis, chronic cough, recurrent pneumonia, hoarseness and dental lesions.

When setting forth a therapeutic plan for treatment of GERD it is suitable to keep in mind that in about 50% of cases said disease becomes chronic, so that it is necessary to individuate a short-term objective in order to solve the acute phase of the illness, and a long-term objective for proper management of the keeping phase (Ruggeri, D., 2002, Doctor, 15:10-15). As far as the pharmacological approach is concerned, various active compounds are employed in treating oesophageal reflux. Drugs usually employed vary from the anti-acid and the H2-antagonistic compounds, which are used in treatment of ulcer, to the most specific ones like the inhibitors of protonic pump (PPI) and the so-called prokinetic compounds, which accelerate the effect of stomach emptying by stimulating the gastric musculature.

WO00/67799 discloses compositions having improved properties comprising an alginate, xanthan gum and/or a carageenan gum, simethicon and sodium bicarbonate in order to treat oesophageal reflux.

It is an object of this invention to individuate a composition against the oesophageal reflux of a mechanical-action type, without undesired secondary effect.

This has been attained by providing a composition in the form of a syrup whose components act through five basic mechanisms:
- reduction of air bubble surface tension which are formed inside the stomach in order to limit the number of bubbles and their propelling action;
- formation of a barrier at the cardiac sphincter level in order to avoid reflux and lower the emission of acid vapors;
- neutralization of gastric juice and vapors;
- creation of a protective barrier for epithelia and induction of epithelial reparative processes;
- reduction of cough.

Thus a composition in the form of a syrup has been identified which contains as the active components magnesium alginate, dimethicone and/or simethicone, carboxypolymethylene (carbomer), dexpanthenol, zinc oxide, and compounds capable of reducing the cough stimulus slightly, as for instance Althaea officinalis, Papaver rhoeas (field poppy) and honey. More particularly:
- magnesium alginate in contact with the acid in the stomach precipitates in the form of a gel of alginic acid. At the same time the reaction between bicarbonate and hydrochloric acid liberates carbon anhydride which by penetrating the net-like structure of the gel makes it to float. In this way a floating mass is formed on chewed food which prevents it from reaching up to the cardiac sphincter level so blocking regurgitation. Moreover it also exerts a buffering action that neutralizes the acid reflux;
- dimethicone (dimethylpolyoxilane) and/or simethicone (a mixture of dimethylpolyoxilanes) are silicone compounds which are inert and hydrorepellent, in liquid form, and are capable of lowering the surface tension of air bubbles which are formed during digestive processes, so as to make said bubbles to coalesce and thus reducing regurgitation and reflux,
- carboxypolymethylene (carbomer) is a viscosity increasing agent which allows syrup to form a protective barrier for epithelia so facilitating their regenerative and/or reparative processes which are induced by re-epithelization compounds (dexpanthenol, and zinc oxide),
- dexpanthenol itself can increase gastric peristalsis so promoting the emptying of stomach,
- Althaea officinalis, Papaver rhoeas (field poppy) and honey, due to their emollient, decongestive and expectorant properties are able to reduce the cough stimulus.

Accordingly, it is an object of this invention a medical device exerting an anti-reflux oesophageal mechanical action, said device having the following composition as weight/volume (g/l) per one litre of syrup:

| | |
|---|---|
| Dimethicone and/or simethicone | 3,3 - 9,9 g |
| Magnesium alginate | 22,5 - 67,5 g |
| Carboxypolymethylene (carbomer) | 0,1 - 0,3 g |
| Dexpanthenol | 0,5 - 1,5 g |
| Zinc oxide | 0,37 - 1,12 g |
| Althaea officinalis | 12,5 - 37,5 g |
| Papaver rhoeas (field poppy) | 12,5 - 37,5 g |
| Honey | 35 - 105 g |

To said composition are also added as eccipients and conserving agents: sodium bicarbonate, sodium hydroxide, sodium methyl p-oxybenzoate, sodium propyl p-oxybenzoate, erythrosine (E127), flavours and fructose.

According to a preferred embodiment said medical device exerting an anti-reflux oesophageal mechanical action has the following composition as weight/volume (g/l) per litre of syrup:

| | |
|---|---|
| Dimethicone and/or simethicone | 6,6 g |
| Magnesium alginate | 45 g |
| Carboxypolymethylene (carbomer) | 0,2 g |
| Dexpanthenol | 1 g |
| Zinc oxide | 0,75 g |
| Althaea officinalis (fluid extract) | 25 g |
| Papaver rhoeas (fluid extract) | 25 g |
| Honey | 70 g |
| Sodium bicarbonate | 0,25 g |
| Sodium hydroxide | 0,66 g |
| Sodium methyl p-oxybenzoate | 1 g |
| Sodium propyl p-oxybenzoate | 0,5 g |
| Erythrosine (E127) | 0,05 g |
| Pomgranate flavour | 2,2 g |
| Strawberry vine flavour | 0,22 g |
| Fructose | 300 g |
| Deionized water | q.s. to 1 litre |

More particularly said syrup is prepared according to the following indications:
Honey, dexpanthenol and fructose are dissolved at 30°C in a deionized water volume 2/3 the total volume; when solubilization is complete, the other components are added sequentially and keeping the solution under continuous mechanical stirring so as to ensure total dispersion of the components, according to the following order:
- the carbomer
- sodium hydroxide
- sodium methyl p-oxibenzoate
- sodium propyl p-oxibenzoate
- magnesium alginate
- fluid extract of Althaea officinalis
- fluid extract of Papaver rhoeas
- zinc oxide
- dimethicone and/or simethicone
- sodium bicarbonate
- erythrosine
- flavours

When a perfect homogeneity of the product is reached, the total volume is checked for a value of one litre, and in case it is less than one litre further deionized water is added to adjust it to the volume. Finally, the pH value of the product is checked, which is to be in the range from 8.3 and 8.4, and its value is adjusted if necessary by adding a 10% solution of sodium hydroxide, the specific weight of the solution being also checked which is to be 1.145.

## Claims

1. An oesophageal anti reflux composition in the form of a syrup comprising means to form a physical barrier at the cardiac sphincter level, **characterized in that** said composition comprises as weight/volume (g/l per one litre of syrup:
| | |
|---|---|
| Dimethicone and/or simethicone | 3,3 - 9,9 g |
| Magnesium alginate | 22,5 - 67,5 g |
| Carboxypolymethylene (carbomer) | 0,1 - 0,3 g |
| Dexpanthenol | 0,5 - 1,5 g |
| Zinc oxide | 0,37 - 1,12 g |
| Althaea officinalis | 12,5 - 37,5 g |
| Papaver rhoeas (field poppy) | 12,5 - 37,5 g |
| Honey | 35 - 105 g |

2. An oesophageal anti-reflux composition in the form of syrup according to claim 1 **characterized in that** said composition comprises as weight/volume (g/l) per one litre of syrup:
| | |
|---|---|
| Dimethicone and/or simethicone | 6, 6 g |
| Magnesium alginate | 45 g |
| Carboxypolymethylene (carbomer) | 0,2 g |
| Dexpanthenol | 1 g |
| Zinc oxide | 0, 75 g |
| Althaea officinalis (fluid extract) | 25 g |
| Papaver rhoeas (fluid extract) | 25 g |
| | |
|---|---|
| Honey | 70 g |
| Sodium bicarbonate | 0, 25 g |
| Sodium hydroxide | 0,66 g |
| Sodium methyl p-oxybenzoate | 1 g |
| Sodium propyl p-oxybenzoate | 0, 5 g |
| Erythrosine (E127) | 0, 05 g |
| Pomgranate flavour | 2,2 g |
| Strawberry vine flavour | 0,22 g |
| Fructose | 300 g |
| Deionized water | q.s. to 1 litre |

## Patentansprüche

1. Zusammensetzung gegen Sodbrennen in Form eines Sirups, umfassend Mittel zum Bilden einer physikalischen Barriere am unteren Ösophagussphinkter, **dadurch gekennzeichnet, dass** die Zusammensetzung - angegeben in Gewicht/Volumen (g/l) - pro einem Liter Sirup enthält:
| | |
|---|---|
| Dimethicon und/oder Simethicon | 3,3 - 9,9 g |
| Magnesiumalginat | 22,5 - 67,5 g |
| Carboxypolymethylen (Carbomer) | 0,1 - 0,3 g |
| Dexpanthenol | 0,5 - 1,5 g |
| Zinkoxid | 0,37 - 1,12 g |
| Althaea officinalis | 12,5 - 37,5 g |
| Papaver rhoeas (Feldmohn) | 12,5 - 37,5 g |
| Honig | 35 - 105 g |

2. Zusammensetzung gegen Sodbrennen in Form eines Sirups nach Anspruch 1, **dadurch gekennzeichnet, dass** die Zusammensetzung - angegeben in Gewicht/Volumen (g/l) - pro einem Liter Sirup enthält:
| | |
|---|---|
| Dimethicon und/oder Simethicon | 6,6 g |
| Magnesiumalginat | 45 g |
| Carboxypolymethylen (Carbomer) | 0,2 g |
| Dexpanthenol | 1 g |
| Zinkoxid | 0,75 g |
| Althaea officinalis (Fluidextrakt) | 25 g |
| Papaver rhoeas (Fluidextrakt) | 25 g |
| Honig | 70 g |
| Natriumbicarbonat | 0,25 g |
| Natriumhydroxid | 0,66 g |
| Natriummethyl-p-oxybenzoat | 1 g |
| Natriumpropyl-p-oxybenzoat | 0,5 g |
| | |
|---|---|
| Erythrosin (E127) | 0,05 g |
| Granatapfelaroma | 2,2 g |
| Erdbeerweinaroma | 0,22 g |
| Fructose | 300 g |
| Deionisiertes Wasser | q.s. auf 1 Liter |

## Revendications

1. Composition anti-reflux gastro-oesophagien se présentant sous la forme d'un sirop comprenant des moyens pour former une barrière physique au niveau du cardia, **caractérisée en ce que** ladite composition comprend les substances suivantes en poids/volume (g/l) par litre de sirop :
| | |
|---|---|
| Diméthicone et/ou siméthicone | 3,3 - 9,9 g |
| Alginate de magnésium | 22,5 - 67,5 g |
| Carboxypolyméthylène (carbomère) | 0,1 - 0,3 g |
| Dexpanthénol | 0,5-1,5 g |
| Oxyde de zinc | 0,37-1,12 g |
| Guimauve officinale (*Althaea officinalis*) | 12,5 - 37,5 g |
| Coquelicot (*Papaver rhoeas*) | 12,5 - 37,5 g |
| Miel | 35 - 105 g |

2. Composition anti-reflux gastro-oesophagien se présentant sous la forme d'un sirop selon la revendication 1, **caractérisée en ce que** ladite composition comprend les substances suivantes en poids/volume (g/l) par litre de sirop :
| | |
|---|---|
| Diméthicone et/ou siméthicone | 6,6 g |
| Alginate de magnésium | 45 g |
| Carboxypolyméthylène (carbomère) | 0,2 g |
| Dexpanthénol | 1 g |
| Oxyde de zinc | 0, 75 g |
| *Althaea officinalis* (extrait liquide) | 25 g |
| *Papaver rhoeas* (extrait liquide) | 25 g |
| Miel | 70 g |
| Bicarbonate de sodium | 0,25 g |
| Hydroxyde de sodium | 0,66 g |
| Méthylparahydroxybenzoate de sodium | 1 g |
| Propylparahydroxybenzoate de sodium | 0,5 g |
| Erythrosine (E127) | 0, 05 g |
| Arôme de grenade | 2,2 g |
| Arôme de vigne fraise | 0,22 g |
| Fructose | 300 g |
| Eau déminéralisée | en quantité suffisante jusqu'à 1 litre |
